# EUROPEAN PATENT APPLICATION

(11) **EP 3 345 896 A1**
(43) Date of publication of application: **11.07.2018**
(21) Application number: 16840821.9
(22) Date of filing: 31.08.2016
(51) Int. Cl.: C07D 307/93, A61K 31/365, A61P 25/28

(54) **DERIVATIVE OF INULA LINEARIIFOLIA LACTONE A**

(30) Priority: 31.08.2015 CN 201510549327
(71) Applicant: Shanxi Zhendong Leading Biotechnology Co., Ltd., Jinzhong, Shanxi 030602 (CN)
(72) Inventor: ZHANG, Weidong, Jinzhong Shanxi 030602 (CN); SUN, Qingyan, Jinzhong Shanxi 030602 (CN); LI, Minghua, Jinzhong Shanxi 030602 (CN); YANG, Baohua, Jinzhong Shanxi 030602 (CN)
(74) Representative: Keil & Schaafhausen Patent- und Rechtsanwälte PartGmbB
(86) International application number: PCT/CN2016/097481
(87) International publication number: WO 2017/036392

(57) **Abstract**

A derivative of inula lineariifolin lactone A.and in particular.A. dimethylamino 4-O-acetyl- Line leaves Inula flowers lactones A or a salt thereof preparation for same, and an application of same in preparation of medicine for treating multiple sclerosis.The dimethylamino 4-O-acetyl-Line leaves Inula flowers lactones A compound is represented by formula I :

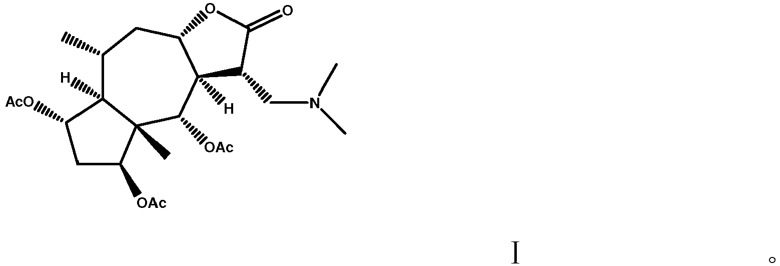

## Description

### Technical field

This invention is involved in medicinal chemistry, typically involved in the preparation and application of Dimethylamino 4-O-acetyl- Line leaves Inula flowers lactones A and its salts in pharmaceuticals, especially involved in the preparation and application of Dimethylamino 4-O-acetyl-Line leaves Inula flowers lactones A and its salts in the treatment for multiple sclerosis.

### Technical background

Since it was first proposed by the French doctor Charcot in 1868, multiple sclerosis (MS) is a disease that still extremely difficult to treat in the world. It is a chronic autoimmune disease that causes physical disabilities by destroying myelin in the brain, spinal cord and optic nerve, and affecting nerve transmission. Because of its high relapsing and disability rate, chronic course and tendency to be developed in young people, usually in thirties, MS has become a common and important nervous system disease that affect the life quality of young people.

No curative therapies for MS are currently available. The goals of therapy with disease-modifying agents in patients with MS include shortening the duration of acute exacerbations, decreasing their frequency, and providing symptomatic relief. It is common practice to treat acute relapses of MS with a short course (typically 3 to 5 days) of a corticosteroid. Thirteen FDA-approved therapeutic agents can reduce disease activity and progression in patients with relapsing forms of MS, including IFN-β, glatiramer acetate(GA), Natalizumab and three oral drugs. Single target drugs such as, GA and are not only expensive but also resistance prone. In addition, intravenous or subcutaneous administration is inconvenient to long-term use. As to the approved three oral drugs, all have severe side effects such as Influenza infection for Gilenya, disturbances of bone marrow function for Aubagio, progressive multifocal leukoencephalopathy for Tecfidera. It is important to investigate more convenient and safer new therapeutic agents for MS.

Traditional Chinese medicines have the characteristics of multiple targets and the action of system adjustment, which show certain medication advantages in complexity and refractory disease such as MS.

Line leaves Inula flowers lactones A (LA) was extracted from Inula lineariifolia Turcz. (syn. Inula linariaefolia) and has good therapeutic effect on experimental autoimmune encephalomyelitis (EAE) model that is a well established animal model for investigation of therapeutic agents for MS. The inventors had applied for patents (A application of LA in the preparation of medicines for multiple sclerosis) in China (CN 20120208157.4) and US(US 2015010548A1) . In order to improve the bioavailability of LA, dimethylamino 4-O-acetyl-LA was synthesized. Lactone ring in weak alkaline environment would be hydrolyzed into carboxyle which would react with the free 4-hydroxyl to form a new lactone ring. So the 4-hydroxyl of LA was acetylized. α-methylene-γ-lactone could react with -SH groups of free cysteine irreversibly, which remarkably decrease the oral bioavailability of LA. Dimethylamino could react with α-methylene through Michael addition reaction to form dimethylamino derivative of 4-O-acetyl LA, which could return to α-methylene in weak alkaline environment. Due to the better solubility, the salts of dimethylamino 4-O-acetyl LA would improve the bioavailability to a better extend.

Experiment results revealed that dimethylamino 4-O-acetyl LA and its salt had good effects on EAE model. Furthermore, they have higher bioavailability than LA. All of these implied their potential applications in the treatment of MS.

### The invention content

The invention aimed at improving the pharmacokinetics profiles of LA mentioned above and preparing derivatives of LA for the treatment of MS.

The invention provides a derivative of LA, dimethylamino 4-O-acetyl LA (compound I).

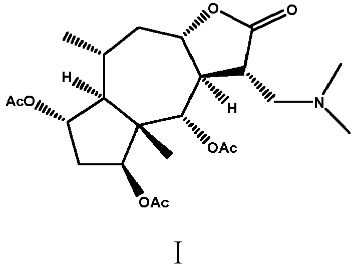

The invention also provides the salts of compound I , synthesized by compound I and acids (HX) applicable in pharmaceuticals. They have the common structure (II) described as below:

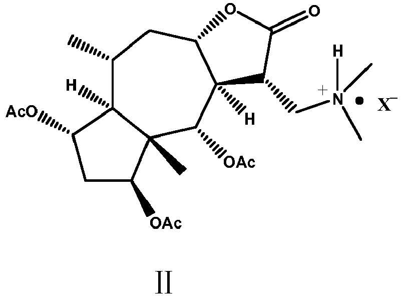

The acids (HX) reacted with compound I could be hydrochloric acid (HCl), sulfuric acid (H₂SO₄), hydrofluoric acid (HF), hydrobromic acid (HBr), hydroiodic acid (HI), Phosphoric acid (H₃PO₄), sulfurous acid (H₂SO₃), nitric acid(HNO₃), carbonic acid (H₂CO₃), boric acid (H₃BO₃), seleninic acid (H₂SeO₃), Phosphomolybdic acid(H₃PO4·12MoO₃), phosphorous acid(H₃PO₃), citric acid, maleic acid, D-malic acid, L-malic acid, DL-malic acid, D-lactic acid, L- lactic acid, DL- lactic acid, oxalic acid, sulphonic acid, benzene sulfonic acid, substituted benzene sulfonic acids, 1- naphthalenesulfonic acid, 2- naphthalenesulfonic acid, phthalic acid, tartaric acid, propandioic acid, succinic acid, fumaric acid, orotic acid, benzoic acid or substituted benzoic acids.

The optimization of the salts of compound I , synthesized by compound I and acids (HX) applicable in pharmaceuticals, could be hydrochloride (compound III), fumarate (compound IV), nitrate (compound V), hydrobromate (compound VI), sulfurate (compound VII), orotate (compound VIII), citrate (compound IX), p-toluenesulfonate (compound X) or succinate (compound XI), especially hydrochloride (compound III) and fumarate (compound IV).

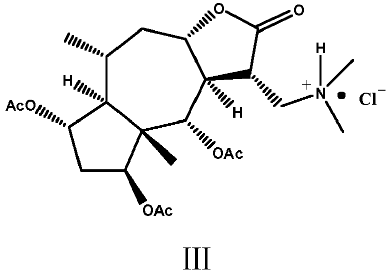

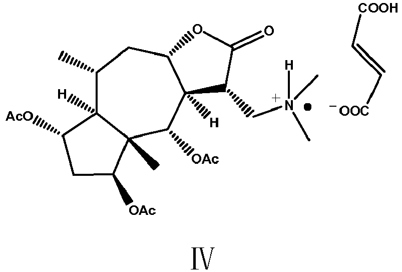

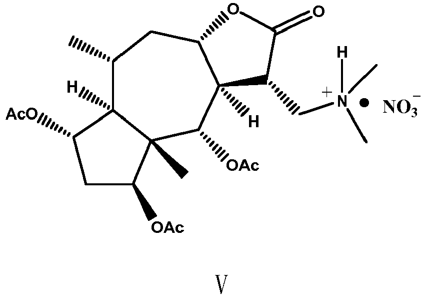

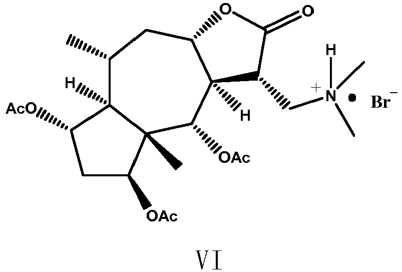

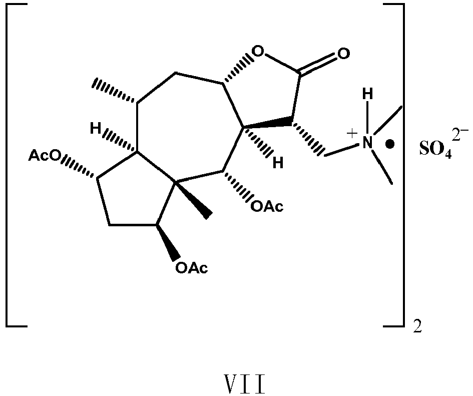

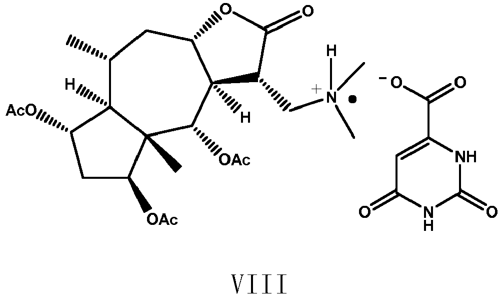

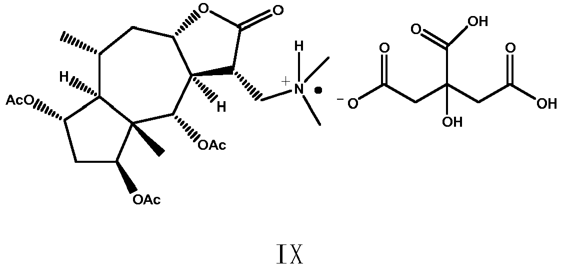

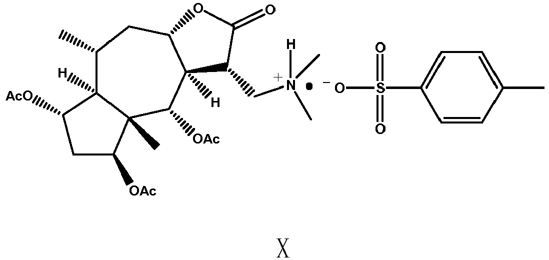

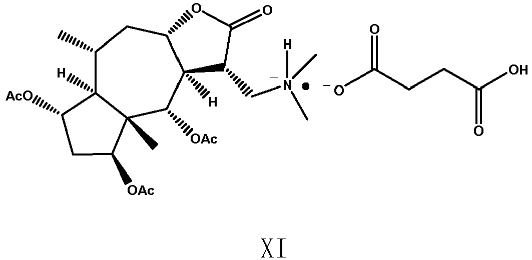

The invention further provides the preparation methods of compound I and its salts that are applicable in pharmaceuticals.

( 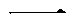 ) The processes to prepare 4-O-acetyl LA were as follows:
(1) The solution of LA, base and acetic anhydride (Ac2O) in the round-bottom flask was stirred at 0∼50°C for 12∼24h, the reaction was monitored by TLC, until the reaction material was consumed completely. The reaction was quenched by water, and the organic layer was retained.
(2) The organic layer was extracted with water several times until the reaction product 4-O-acetyl LA in the organic phase was pure without other impurities (monitored by TLC), then the aqueous layer was discarded. The organic layer was washed with saturated brine and dried with anhydrous sodium sulfate. Then the solvent was evaporated to 1/10∼1/20 of the original volume under reduced pressure (0.01∼0.1MPa) at 20∼80 °C.
(3) The concentrated solution was purified by silica-gel column chromatography, gradient eluted with PE/EA 15:1∼5:1 to afford 4-O-acetyl LA, monitored by TLC.
   Represented as the following equation: 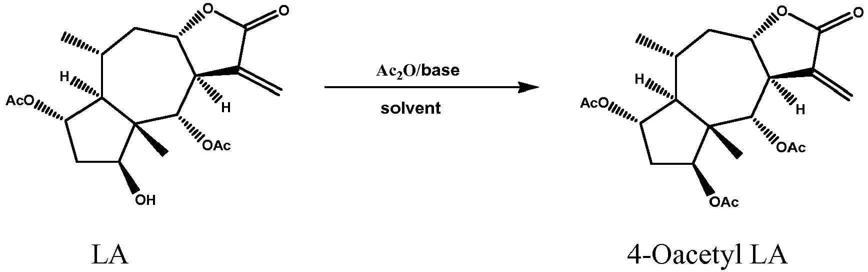
(4) The solution of 4-O-acetyl LA and dimethylamine hydrochloride in the reaction flask was stirred at -20∼0°C, the base was added to the mixture slowly and carefully, then the reaction mixture was warmed up to room temperature (25-30°C) and stirred for 2∼12h, the reaction was monitored by TLC. Until the reaction material was consumed completely, the solvent was evaporated under reduced pressure (0.01∼0.1MPa) at 20∼80 °C to afford the rude product. Then the rude product was dissolved in the organic solvent, extracted with water, the aqueous layer was discarded, the same procedure was repeated several times until the reaction product dimethylamino-4-O-acetyl LA in the organic layer was pure without other impurities (monitored by TLC), The organic layer was washed with saturated brine and dried with anhydrous sodium sulfate. The concentrated solution was purified by silica-gel column chromatography, gradient eluted with PE/EA 15:1∼1:1 to afford the solution of dimethylamino-4-O-acetyl LA, monitored by TLC. Then the solvent was evaporated under reduced pressure (0.01∼0.1MPa) at 20∼80 °C to afford dimethylamino-4-O-acetyl LA (compound I)

Represented as the following equation:

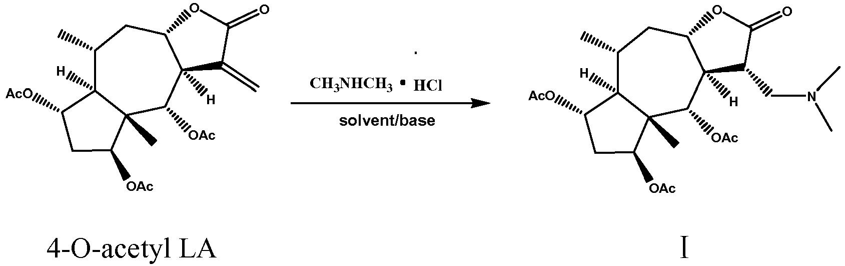

The preparation method of dimethylamino-4-O-acetyl LA was characterized in that the ratio of LA, base and acetic anhydride (Ac₂O) was 1.0eq: 1.0∼2.0eq: 1.0∼2.0eq in the first step.

The mentioned organic reagent was one or several mixtures of ethyl acetate, dichloromethane, chloroform, tetrachloromethane, diethyl ether, petroleum ether, n-butyl alcohol, benzene, methylbenzene or tetrahydrofuran. Dichloromethane was preferred.

The mentioned base was low molecular weight trialkyl amine, which was selected from trimethylamine, triethylanmine, tripropylamine, tributylamine, pyridine or replaced pyridine. 2,6-Dimethylpyridine was preferred.

The ratio of 4-O-acetyl LA, base and acetic anhydride (Ac₂O) was 1.0eq: 1.0∼2.0eq: 1.0∼2.0eq in the fourth step. The mentioned organic solvent was one or several mixtures of low molecular weight fatty alcohols, which was selected from methanol, ethanol, propanol, isopropanol, dichloromethane, chloroform, tetrachloromethane, diethyl ether, petroleum ether or ethyl acetate. Ethanol was preferred.

The mentioned organic reagent was one or several mixtures of ethyl acetate, dichloromethane, chloroform, tetrachloromethane, diethyl ether, petroleum ether, n-butyl alcohol, benzene, methylbenzene or tetrahydrofuran. Dichloromethane was preferred.

The mentioned base was low molecular weight trialkylamine, pyridine or the replaced pyridine. The alkyl groups were methyl, ethyl, propyl and butyl. Triethylamine was preferred.

( 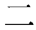 ) The preparation method of salt of dimethy-4-O-acetyl LA was as follows:
The solution of compound I and acid (HX) was stirred at 0∼50°C for 2∼24h, then the solvent was evaporated under reduced pressure (0.01∼0.1MPa) at 20∼80°C to afford the salt of compound II
Represented as the following equation:

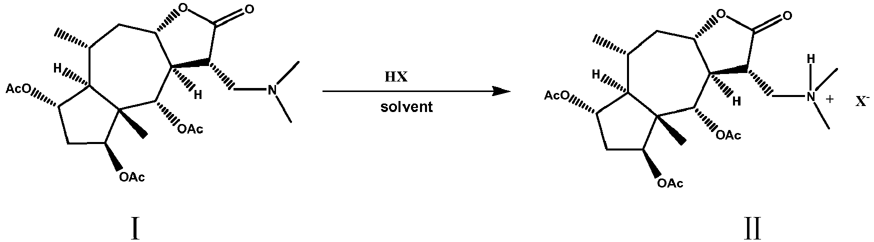

The ratio of dimethy-4-O-acetyl LA (compound I) and acid (HX) was 1.0eq: 1.0∼1.5eq in the above method.
The mentioned HX was hydrochloric acid (HCl), sulfuric acid (H₂SO₄), hydrofluoric acid (HF), hydrobromic acid (HBr), hydroiodic acid (HI), Phosphoric acid (H₃PO₄), sulfurous acid (H₂SO₃), nitric acid(HNO₃), carbonic acid (H₂CO₃), boric acid (H₃BO₃), seleninic acid(H₂SeO₃), Phosphomolybdic acid(H₃PO4·12MoO₃), phosphorous acid(H₃PO₃), citric acid, maleic acid, D-malic acid, L-malic acid, DL-malic acid, D-lactic acid, L- lactic acid, DL- lactic acid, oxalic acid, sulphonic acid, benzene sulfonic acid, substituted benzene sulfonic acids, 1-naphthalenesulfonic acid, 2- naphthalenesulfonic acid, phthalic acid, tartaric acid, propandioic acid, butanedioic acid, fumaric acid, orotic acid, benzoic acid or substituted benzoic acids. hydrochloric acid and fumaric acid were preferred.

The mentioned solvent was one or several mixtures of low molecular weight fatty alcohols, which was selected frommethanol, ethanol, propanol, isopropanol, dichloromethane, chloroform, tetrachloromethane, diethyl ether, petroleum ether or ethyl acetate. Ethanol was preferred.

The invention also provides crystal forms A, D, F, G of compund IV and their preparation methods.

The mentioned crystal form A of compound IV was characterised using PXRD (powder X-ray diffraction) with the values of 2θ, d and relative hights listed in Table 1.

**Table 1 PXRD data of compund IV form A**

| 2θ | d(Ǻ) | I(Height)% |
|---|---|---|
| 8.101 | 10.9048 | 5.6 |
| 9.72 | 9.092 | 66.5 |
| 10.22 | 8.6484 | 77.4 |
| 11.62 | 7.6093 | 37.7 |
| 12.381 | 7.1435 | 6.8 |
| 14.86 | 5.9568 | 11.7 |
| 15.84 | 5.5903 | 37.8 |
| 16.28 | 5.4402 | 32.3 |
| 17.499 | 5.0639 | 18.4 |
| 19.56 | 4.5347 | 70.2 |
| 20.64 | 4.2998 | 100 |
| 21.081 | 4.2108 | 21.9 |
| 21.819 | 4.07 | 42.6 |
| 23.381 | 3.8016 | 72.4 |
| 24.12 | 3.6866 | 4.3 |
| 24.939 | 3.5674 | 41.5 |
| 25.281 | 3.52 | 40.3 |
| 26.34 | 3.3808 | 7.5 |
| 26.901 | 3.3115 | 11.4 |
| 27.581 | 3.2315 | 16.4 |
| 29.436 | 3.0318 | 4.8 |
| 30.021 | 2.9741 | 7.2 |
| 31.001 | 2.8823 | 23.4 |
| 32.038 | 2.7913 | 6.9 |
| 32.498 | 2.7528 | 2.3 |
| 32.922 | 2.7183 | 3.2 |
| 33.302 | 2.6882 | 10.1 |
| 34.361 | 2.6077 | 3.2 |
| 35.879 | 2.5008 | 5.9 |
| 37.34 | 2.4062 | 5.5 |
| 38.998 | 2.3077 | 4.3 |
| 40.121 | 2.2456 | 7.5 |
| 41.8 | 2.1592 | 7.4 |

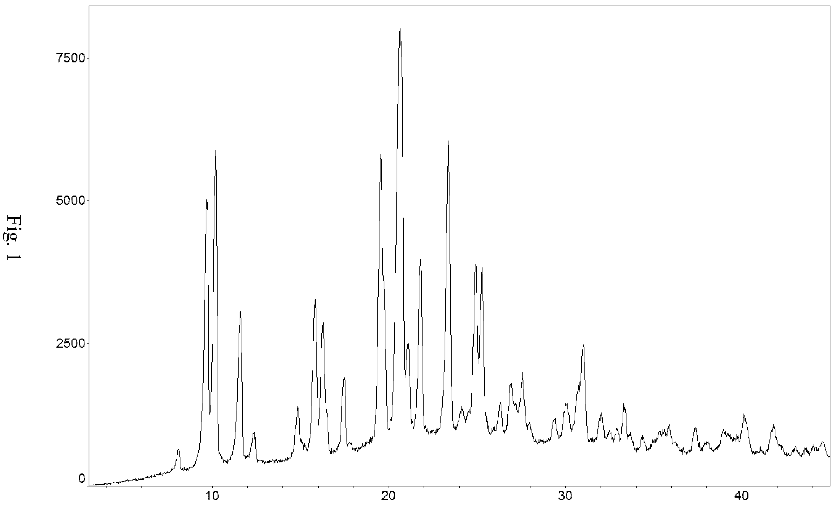

The PXRD pattern of form A was shown in Fig. 1.

Form A could be prepared as described next. First, compound IV was dissolved in 2 to 5 folds ethyl acetate. Then 2 to 5 folds hexane were added and stirred until a lot of crystals deposited. The crystals were filtrated and dried in vacuum at 25∼40°C to get form A of compound IV.

The mentioned crystal form D of compound IV was characterised using PXRD with the values of 2θ, d and relative hights listed in Table 2.

**Table 2 PXRD data of compund IV form D**

| 2θ | d(Ǻ) | I(Height)% |
|---|---|---|
| 8.6 | 10.2736 | 100 |
| 9.777 | 9.0388 | 30.8 |
| 10.659 | 8.2929 | 21.5 |
| 11.102 | 7.9633 | 3.7 |
| 13.021 | 6.7936 | 10 |
| 14.64 | 6.0457 | 60.5 |
| 15.039 | 5.8861 | 24 |
| 16.24 | 5.4535 | 35.4 |
| 16.879 | 5.2484 | 20 |
| 17.298 | 5.1221 | 4.7 |
| 17.8 | 4.979 | 5.5 |
| 18.659 | 4.7514 | 13.4 |
| 19.12 | 4.638 | 16.1 |
| 19.619 | 4.5211 | 78.2 |
| 21.418 | 4.1453 | 10.6 |
| 22.3 | 3.9832 | 42.2 |
| 22.801 | 3.8969 | 10.3 |
| 23.266 | 3.82 | 3.8 |
| 23.939 | 3.7141 | 50.4 |
| 24.699 | 3.6015 | 35.6 |
| 25.259 | 3.5229 | 9.7 |
| 25.639 | 3.4716 | 8.3 |
| 26.24 | 3.3935 | 20.8 |
| 27.24 | 3.2711 | 19 |
| 27.961 | 3.1883 | 8.8 |
| 28.379 | 3.1423 | 4.2 |
| 28.659 | 3.1123 | 15.7 |
| 28.981 | 3.0784 | 12.7 |
| 29.62 | 3.0135 | 14.2 |
| 29.941 | 2.9818 | 6.5 |
| 30.36 | 2.9416 | 11.2 |
| 30.996 | 2.8827 | 7.4 |
| 31.478 | 2.8397 | 4 |
| 31.861 | 2.8064 | 11.5 |
| 35.799 | 2.5062 | 4.6 |
| 36.646 | 2.4502 | 4.7 |
| 39.882 | 2.2586 | 7.3 |
| 41.057 | 2.1966 | 9.5 |
| 41.9 | 2.1543 | 4.5 |

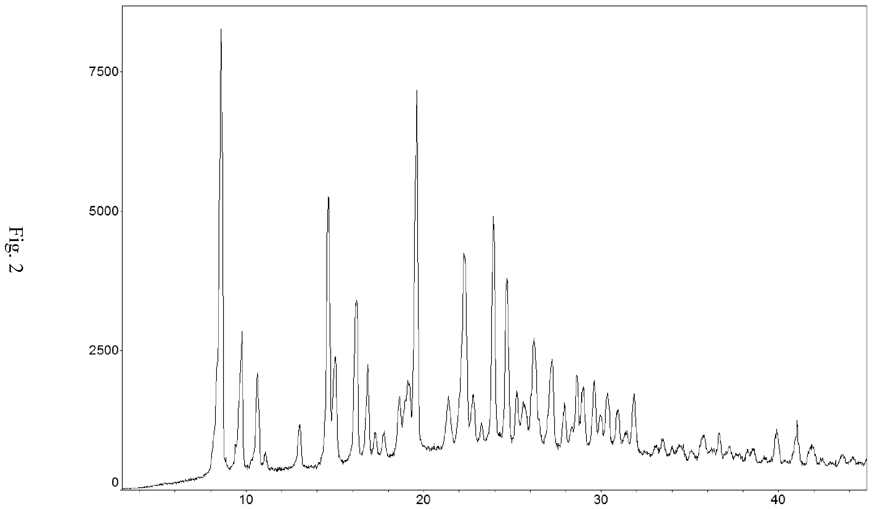

The PXRD pattern of form D was shown in Fig. 2.

Form D could be prepared as described next. Compound IV was put into MTBE (methyl tert-butyl ether) containing 0.2% water, then stirred for 2-5 hours. The solvent was removed by filtration and the solid was dried at 40-60°C under 0.03-0.1MPa for 4-24 hours to ger form D of compound IV.

The mentioned crystal form F of compound IV was characterised using PXRD with the values of 2θ, d and relative hights listed in Table 3.

**Table 3 PXRD data of compund IV form F**

| 2θ | d(Ǻ) | I(Height)% |
|---|---|---|
| 8.06 | 10.9609 | 5.4 |
| 9.659 | 9.1493 | 100 |
| 10.08 | 8.7682 | 91.7 |
| 11.5 | 7.6884 | 42.3 |
| 12.139 | 7.2851 | 7.7 |
| 14.642 | 6.0449 | 9.8 |
| 15.639 | 5.6616 | 35.8 |
| 16.2 | 5.4669 | 30 |
| 17.441 | 5.0806 | 15.1 |
| 19.48 | 4.5531 | 73 |
| 20.44 | 4.3413 | 68.1 |
| 20.679 | 4.2916 | 99.4 |
| 21.639 | 4.1034 | 40.1 |
| 23.28 | 3.8177 | 54.9 |
| 24.68 | 3.6043 | 21.8 |
| 25.261 | 3.5227 | 29.8 |
| 25.938 | 3.4322 | 6 |
| 26.52 | 3.3582 | 11.3 |
| 26.938 | 3.3071 | 4.3 |
| 27.482 | 3.2428 | 9.8 |
| 27.958 | 3.1887 | 4.4 |
| 29.201 | 3.0557 | 5.1 |
| 29.918 | 2.9841 | 7.3 |
| 30.442 | 2.9339 | 8.6 |
| 30.839 | 2.8971 | 13.4 |
| 31.657 | 2.824 | 4.7 |
| 33.3 | 2.6884 | 6.2 |
| 34.341 | 2.6092 | 2.6 |
| 35.02 | 2.5601 | 4.1 |
| 35.778 | 2.5076 | 4.3 |

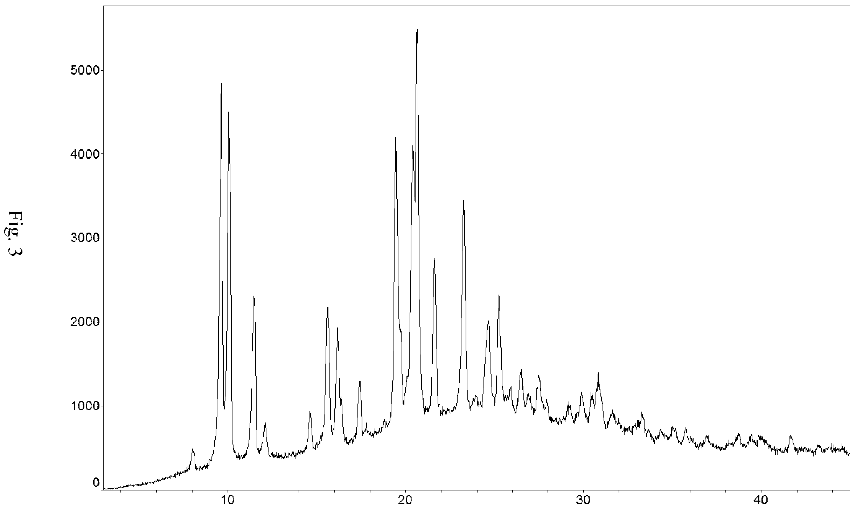

The PXRD pattern of form F was shown in Fig. 3.
Form F could be prepared as described next. compound IV was dissolved in 1 to 2 folds methanol. Then 2 to 5 folds ether were added and stirred until a lot of crystals deposited. The crystals were filtrated and dried at 25∼40°C under 0.03-0.1MPa to get form F of compound IV.

The mentioned crystal form G of compound IV was characterised using PXRD with the values of 2θ, d and relative hights listed in Table 4.

**Table 4 PXRD data of compund IV form G**

| 2θ | d(Ǻ) | I(Height)% |
|---|---|---|
| 8.567 | 10.31279 | 100.0 |
| 7.931 | 9.08148 | 17.0 |
| 10.849 | 8.14803 | 16.7 |
| 11.163 | 7.92006 | 193.4 |
| 12.452 | 7.10295 | 0.8 |
| 12.865 | 6.87584 | 2.7 |
| 13.115 | 6.74542 | 1.0 |
| 14.413 | 6.14053 | 17.9 |
| 14.869 | 5.95311 | 20.0 |
| 15.128 | 5.85184 | 3.2 |
| 16.225 | 5.45870 | 9.0 |
| 17.268 | 5.13128 | 13.8 |
| 18.761 | 4.72601 | 26.8 |
| 19.661 | 4.51181 | 6.0 |
| 21.872 | 4.06030 | 20.3 |
| 22.059 | 4.02635 | 22.8 |
| 23.059 | 3.85401 | 1.9 |
| 23.631 | 3.73193 | 3.0 |
| 24.014 | 3.70281 | 2.1 |
| 24.809 | 3.58595 | 3.0 |
| 25.401 | 3.50361 | 13.1 |
| 25.830 | 3.44652 | 6.9 |
| 26.245 | 3.39285 | 7.6 |
| 27.267 | 3.26804 | 2.5 |
| 28.160 | 3.16634 | 5.4 |
| 29.131 | 3.06300 | 5.0 |
| 30.138 | 2.96286 | 3.8 |
| 30.797 | 2.90094 | 4.1 |
| 32.824 | 2.72631 | 1.0 |
| 33.257 | 2.69179 | 0.6 |
| 34.654 | 2.58642 | 1.6 |
| 36.124 | 2.48445 | 1.2 |
| 38.027 | 2.36438 | 1.0 |
| 38.503 | 2.33628 | 1.0 |
| 38.942 | 2.31091 | 1.0 |

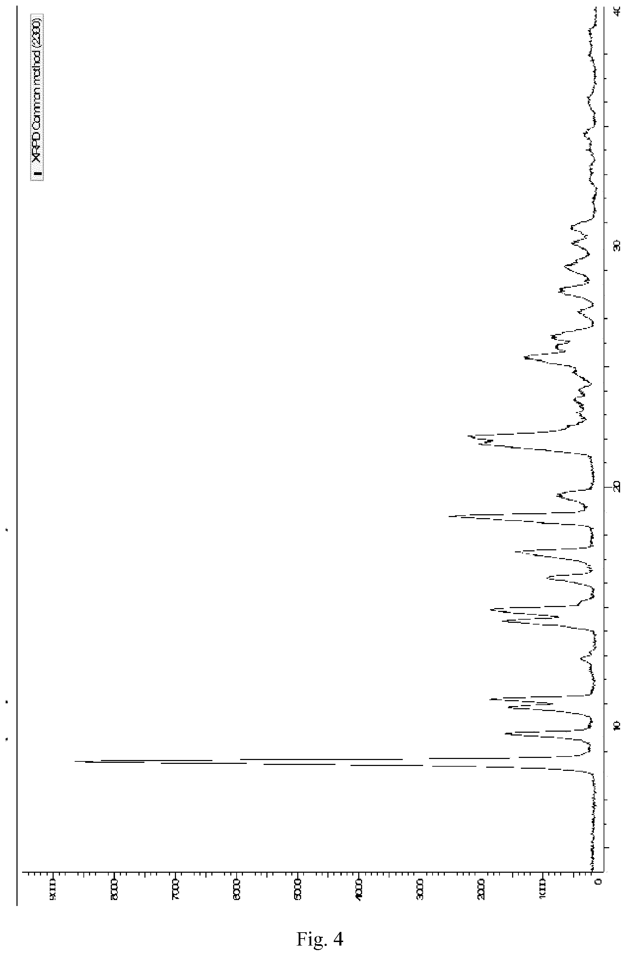

The PXRD pattern of form G was shown in Fig. 4.

Form G could be prepared as described next. Compound I was dissolved in 2 folds ethanol, then added with equivalent fumaric acid when stirring. The mixture was stirred for 0.5-2 hours and then added with 1 to 2 folds MTBE to seperate out crystals. The crystals were filtrated , drip washed by MTBE and dried 25∼40°C in vacuum to get form G of compund IV.

The invention finally provides the application of compound I and its salts in the treatment of MS.

Experimental autoimmune encephalomyelitis (EAE) is a well established animal model that still is the most widely accepted animal model for the study of the pathogenesis of MS and also serves as a valuable tool for testing new therapies of MS. Compound 1 and 3 showed good effects to EAE mice after oral administration from day 0 to day 31 after immunization, which indicated that compound 1 or its salt could be used in the preparation of drugs for the treatment of MS.

The drugs prepared for MS mentioned in the invention are composed of compound I or its salts as the only active ingredient, combined with medicinal carriers. Preparations could be tablets, dispersible tablets, mouth collapse tablets, retard tablets, capsules, soft capsules, dropping pills, granules, injections, powder injections or aerosols.

Dimethylamino 4-O-acetyl LA and its salts, provided in the invention, have higher bioavailability than LA. The invention provides new potential clinical treatments for MS and has good social benefit.

### Description of the attached figures

Figure 1 PXRD pattern of compund IV form A.
Figure 2 PXRD pattern of compund IV form D.
Figure 3 PXRD pattern of compund IV form F.
Figure 4 PXRD pattern of compund IV form G.
Figure 5 Clinical course of EAE after orally administrated compound I (30mg/kg). Y-axis is clinical score and X-axis is days after immunization (Three lines from top to bottom represented EAE model treated by vehicle, EAE model treated by compound I and naive, respectively).
Figure 6 Clinical course of EAE after orally administrated compound IV (15mg/kg). Y-axis is clinical score and X-axis is days after immunization (Three lines from top to bottom represented EAE model treated by vehicle, EAE model treated by compound IV and naive, respectively).
Figure 7 H&E analysis of Cervical Spinal Cord (1: naive, 2: EAE model treated by vehicle, 3:model treated by compound I 30mg/kg) and LFB staining of Cervical Spinal Cord (4: naive, 5: EAE model treated by vehicle,6: model treated by compound I 30mg/kg).
Figure 8 H&E analysis of Cervical Spinal Cord (1: naive, 2: EAE model treated by vehicle, 3:model treated by compound IV 15mg/kg) and LFB staining of Cervical Spinal Cord (4: naive, 5: EAE model treated by vehicle,6: model treated by compound IV 15mg/kg).

### Specific implementation examples:

Materials and reagents used in the following examples were purchased from the market.

### Example 1 Preparation of L A

The dried Line leaves Inula flowers grass 50kg was cut into pieces ,then extracted with 750 L ethanol (80 ∼ 95% V/V) for two times, 2 hours each time. The extracted fluid was combined and condensed into liquid extract with a density of 1g raw material per milliliter extract under reduced pressure. The liquid extract was diluted with 50 L water , then extracted 5 times with 50 L petroleum ether each time to get the petroleum ether extract that was condensed and further purified by silica gel column chromatography. The silica gel column was eluted with gradient petroleum ether/ethyl acetate (100:0 ∼ 1:1). The elution was detected by thin-layer chromatography and the portion containing LA was collected and condensed to apply on C18 reverse phase column chromatography for further purification . The C18 column was eluted with gradient methanol/water (50:100∼70:100) and The portion containing only LA was collected and condensed to get 45.3g LA.

The compound was identified first by mass spectrometry to get the molecular weight of 366 and formula of C19H28O7, then by¹ H-NMR and ¹³C-NMR, as well as two-dimensional NMR. The data were consistent with those of LA.

### Example 2 Preparation of 4-O-acetyl-LA

LA (10.0 g, 0.027mol, 1.0 eq), 2,6-Dimethylpyridine (DMAP) (4.0 g, 0.033 mol, 1.2 eq) and acetic anhydride(Ac2O)(3.34 g, 0.033 mol, 1.2 eq) were dissolved in 200mL dichloromethane, then stirred in a 500mL round-bottom flask at 0∼50°C for 12h, the reaction was monitored by TLC, until the reaction material was consumed completely. The reaction was quenched by 200mL water, and the organic layer was retained.

The organic layer was extracted with 100 mL water several times until the reaction product 4-O-acetyl LA in the organic phase was pure without DMPA (monitored by TLC), then the aqueous layer was discarded. The organic layer was washed with saturated brine and dried with anhydrous sodium sulfate. Then the solvent was evaporated to 1/10∼1/20 of the original volume under reduced pressure (0.01∼0.1MPa) at 40°C.

The concentrated solution was purified by silica-gel column chromatography, gradient eluted with PE/EA 15:1∼5:1 to afford 4-O-acetyl LA (10.5 g, 0.026 mol) with a yield of 94%.

The NMR data of the compound: ¹H NMR (500 MHz, Chloroform-d) δ 6.14 (dt, J = 3.8, 1.1 Hz, 1H), 5.51 (dd, J = 3.2, 1.6 Hz, 1H), 5.26 - 5.19 (m, 1H), 5.15 (dt, J = 8.6, 1.6 Hz, 1H), 4.91 (t, J = 7.8 Hz, 1H), 4.49 - 4.42 (m, 1H), 3.01 - 2.93 (m, 1H), 2.43 - 2.36 (m, 1H), 2.08 - 2.06 (m, 3H), 2.03 (q, J = 1.2 Hz, 3H), 2.00 - 1.93 (m, 6H), 1.89 (dd, J = 12.0, 5.9 Hz, 1H), 1.48 - 1.38 (m, 1H), 1.08 (d, J = 1.2 Hz, 3H), 0.97 (dd, J = 6.4, 1.4 Hz, 3H); ¹³C NMR (125 MHz, Chloroform-d) δ 170.7, 170.3, 169.7, 168.9, 137.9, 121.2, 76.1, 75.0, 73.7, 73.6, 52.4, 50.3, 50.3, 43.9, 34.2, 29.8, 21.2, 21.1, 20.9, 20.3, 17.3.

### Example 3 The preparation of dimethyl-4-O-acetyl LA(compound I)

4-O-acetyl LA(10.0 g, 0.024 mol, 1.0 eq) and dimethylamine hydrochloride (CH₃NHCH₃·HCl) (3.0 g, 0.037 mol, 1.5 eq) were dissolved in 200mL ethanol, then stirred in a 500mL reaction flask at 0°C. Triethylamine (5.1 mL, 0.037 mol, 1.5 eq) was added to the mixture slowly and carefully, then the reaction mixture was warmed up to room temperature (25-30°C) and stirred for 4h, the reaction was monitored by TLC. Until the reaction material was consumed completely, the solvent was evaporated under reduced pressure (0.01∼0.1MPa) at 40°C to afford the rude product. Then the rude product was dissolved in 200mL dichloromethane, extracted with water. the aqueous layer was discarded, the same procedure was repeated several times until the reaction product dimethylamino-4-O-acetyl LA in the organic layer was pure without other impurities (monitored by TLC), The organic layer was washed with saturated brine and dried with anhydrous sodium sulfate. The concentrated solution was purified by silica-gel column chromatography, gradient eluted with PE/EA 15:1∼1:1 to afford the solution of dimethylamino-4-O-acetyl LA, monitored by TLC. Then the solvent was evaporated under reduced pressure (0.01∼0.1MPa) at 40°C to afford dimethylamino-4-O-acetyl LA (compound I) (10.0 g, 0.022 mol) with a yield of 90%.

The NMR data of compound I : ¹H-NMR (500 MHz, Chloroform-d) δ 5.27 (dd, J = 10.7, 8.6 Hz, 1H), 5.04 (d, J = 9.5 Hz, 1H), 4.92 (ddd, J = 9.2, 6.8, 2.1 Hz, 1H), 4.40 (ddd, J = 11.7, 10.1, 2.8 Hz, 1H), 2.69 - 2.61 (m, 2H), 2.53 (dd, J = 9.3, 6.5 Hz, 1H), 2.45 (dd, J = 13.1, 5.3 Hz, 1H), 2.36 (ddd, J = 13.2, 4.5, 2.8 Hz, 1H), 2.18 (s, 6H), 2.08 - 2.05 (m, 1H), 2.03 (s, 4H), 2.01 (s, 3H), 1.97 (s, 3H), 1.96 - 1.87 (m, 2H), 1.40 (dt, J = 13.1, 11.9 Hz, 1H), 1.12 (s, 3H), 0.97 (d, J = 6.5 Hz, 3H); ¹³C-NMR (125 MHz, Chloroform-d) δ 176.6, 170.4, 170.3, 169.5, 77.2, 76.2, 76.1, 74.9, 74.3, 57.4, 50.0, 50.0, 48.7, 46.9, 46.3, 43.8, 34.3, 29.4, 21.1, 21.1, 20.8, 20.16, 18.5.

### Example 4 The preparation of compound III

Compound I (10.0g, 0.022 mol) obtained through example 3 was dissolved in 200mL anhydrous methanol, then stirred at room temperature (25-30°C) for half an hour after 500mL standard HCl gas flowing in. The solution was evaporated under reduced pressure (0.01∼0.1MPa) at 40°C to afford the hydrochloride (compound III) (10.0 g, 0.022 mol).

The NMR data of compound III: ¹H-NMR (500 MHz, D₂O) δ 5.24 - 5.16 (m, 2H), 5.02 (td, J = 6.8, 4.5 Hz, 1H), 4.93 - 4.84 (m, 1H), 3.68 (dd, J = 13.4, 11.5 Hz, 1H), 3.45 (td, J = 11.4, 4.0 Hz, 1H), 3.37 (s, 1H), 2.99 (dd, J = 13.3, 4.0 Hz, 7H), 2.74 (d, J = 11.5 Hz, 1H), 2.44 - 2.36 (m, 1H), 2.19 (s, 3H), 2.16(d, J = 6.7 Hz, 1H), 2.14 (d, J = 1.0 Hz, 3H), 2.13 - 2.07 (m, 2H), 2.08 (s, 3H), 1.53 (q, J = 12.2 Hz, 1H), 1.22 (s, 3H), 0.99 (d, J = 6.5 Hz, 3H); ¹³C NMR (125 MHz, D₂O) δ 177.20, 172.43, 171.0 (2×C), 79.2, 77.2 (2×C), 75.9, 75.2, 57.4, 49.8, 49.8(2×C), 49.4, 42.3, 41.9, 33.5, 28.5, 20.9, 20.57, 20.6, 19.5, 17.40.

### Example 5 The preparation of compound IV

Compound I (10.0g, 0.022 mol) obtained through example 3 was dissolved in 200mL anhydrous methanol, added analytical fumaric acid (2.6g, 0.022mol), and then stirred at room temperature (25-30°C) for half an hour. The solution was evaporated under reduced pressure (0.01∼0.1MPa) at 40°C to afford the fumarate (compound IV) (12.6g, 0.022 mol).

The NMR data of compound IV: ¹H-NMR (500 MHz, D₂O) δ 6.72 (d, J = 1.1 Hz, 2H), 5.23 - 5.16 (m, 2H), 5.00 (td, J = 6.8, 4.5 Hz, 1H), 4.90 - 4.84 (m, 1H), 3.66 (dd, J = 13.4, 11.5 Hz, 1H), 3.44 (td, J = 11.4, 4.0 Hz, 1H), 3.36 (s, 1H), 2.98 (dd, J = 13.3, 4.0 Hz, 7H), 2.72 (d, J = 11.5 Hz, 1H), 2.42 - 2.36 (m, 1H), 2.17 (s, 3H), 2.14 (d, J = 6.7 Hz, 1H), 2.12 (d, J = 1.0 Hz, 3H), 2.11 - 2.07 (m, 2H), 2.06 (s, 3H), 1.50 (q, J = 12.2 Hz, 1H), 1.20 (s, 3H), 0.98 (d, J = 6.5 Hz, 3H); ¹³C-NMR (125 MHz, D₂O) δ 177.18, 173.61, 173.50, 172.41, 170.9 (2×C), 134.8 (2×C), 79.0, 77.0 (2×C), 75.7, 75.0, 57.2, 49.6, 49.6(2×C), 49.2, 42.1, 41.7, 33.3, 28.3, 20.7, 20.5, 20.4, 19.3, 17.2.

### Example 6 The preparation of compound V

Compound I (10.00mg, 0.22m mol) obtained through example 3 was dissolved in 10mL ethyl acetate, added 1% nitric acid (1mL, 0.22mmol), and then stirred at room temperature (25-30°C) for half an hour. The solution was evaporated under reduced pressure (0.03∼0.1MPa) at 40°Cto afford the nitrate (compound V) (113mg, 0.22mmol).
¹H NMR (400 MHz, (CD3)2SO) of compound V : 8.92 (s, 1H), 5.03-5.10 (m, 2H), 4.89-4.94 (m, 1H), 4.65-4.71 (m, 1H), 3.46-3.52 (m, 1H), 3.36-3.39 (m, 1H), 2.81-2.87 (m, 4H), 2.73 (s, 3H), 2.63 (q, J = 10.0 Hz, 1H), 2.18-2.21 (m, 1H), 2.05 (s, 3H), 2.02 (s, 3H), 1.83-1.99 (m, 7H), 1.41 (q, J = 11.6 Hz, 1H), 1.09 (s, 3H), 0.89 (d, J = 5.6 Hz, 3H).

### Example 7 The preparation of compound VI

Compound I (10.00mg, 0.22m mol) obtained through example 3 was dissolved in 10mL ethyl acetate, added 1M hydrobromic acid (0.22mL, 0.22mmol), and then stirred at 25°C for half an hour. The solution was evaporated under reduced pressure (0.03∼0.1MPa) at 40°C to afford the hydrobromate (compound VI) (117mg, 0.22mmol).
¹H NMR(400 MHz, (CD3)2SO) of compound VI: 8.96 (br, 1H), 5.01-5.09 (m, 2H), 4.91-4.94 (m, 1H), 4.66-4.72 (m, 1H), 3.32-3.40 (m, 2H), 2.59-2.85 (m, 8H), 2.18-2.21 (m, 1H), 2.05 (s, 3H), 2.02 (s, 3H), 1.83-1.99 (m, 7H), 1.41 (q, J = 12.0 Hz, 1H), 1.10 (s, 3H), 0.89 (d, J = 5.6 Hz, 3H).

### Example 8 The preparation of compound VII

Compound I (10.00mg, 0.22m mol) obtained through example 3 was dissolved in 10mL ethyl acetate, added 1% sulfuric acid (0.6mL, 0.11mmol), and then stirred at 25°Cfor half an hour. The solution was evaporated under reduced pressure (0.03∼0.1MPa) at 40°C to afford the sulfurate (compound VII) (110mg, 0.22mmol).
¹H NMR (400 MHz, (CD3)2SO) of compound VII: 8.92 (s, 1H), 5.01-5.09 (m, 2H), 4.91-4.94 (m, 1H), 4.66-4.72 (m, 1H), 4.52 (br, 1H), 3.47-3.53 (m, 1H), 3.34-3.40 (m, 1H), 2.79-2.88 (m, 4H), 2.73 (s, 3H), 2.63 (q, J = 10.0 Hz, 1H), 2.18-2.21 (m, 1H), 2.05 (s, 3H), 2.02 (s, 3H), 1.83-1.99 (m, 7H), 1.41 (q, J = 11.6 Hz, 1H), 1.10 (s, 3H), 0.89 (d, J = 5.6 Hz, 3H).

### Example 9 The preparation of compound VIII

Compound I (10.00mg, 0.22m mol) obtained through example 3 was dissolved in 10mL ethyl acetate, added orotic acid (35mg, 0.22mmol), and then stirred at 25°C for half an hour. The solution was evaporated under reduced pressure (0.03∼0.1MPa) at 40°C to afford the orotate (compound VII) (135mg, 0.22mmol).
¹H NMR (400 MHz, (CD3)2SO) of compound VII: 11.05 (s, 1H), 9.93 (s, 1H), 5.83 (s, 1H), 5.02-5.07 (m, 2H), 4.90-4.93 (m, 1H), 4.61-4.67 (m, 1H), 3.68 (br, 1H), 3.06-3.14 (m, 2H), 2.79 (d, J = 11.6 Hz, 1H), 2.65 (q, J = 10.0 Hz, 1H), 2.56 (s, 6H), 2.18-2.22 (m, 1H), 2.02 (s, 3H), 2.00 (s, 3H), 1.82-1.98 (m, 7H), 1.38 (q, J = 12.0 Hz, 1H), 1.09 (s, 3H), 0.88 (d, J = 6.0 Hz, 3H).

### Example 10 The preparation of compound IX

Compound I (10.00mg, 0.22m mol) obtained through example 3 was dissolved in 10mL ethyl acetate, added citric acid (43mg, 0.22mmol), and then stirred at 25°Cfor half an hour. The solution was evaporated under reduced pressure (0.03∼0.1MPa) at 40°C to afford the citrate (compound IX) (143mg, 0.22mmol).
¹H NMR(400 MHz, (CD3)2SO) of compound IX: 11.44 (br, 1H), 8.33 (br, 1H), 5.97 (d, J = 3.2 Hz, 1H), 5.35 (d, J = 2.8 Hz, 1H), 5.01-5.10 (m, 2H), 4.91-4.95 (m, 1H), 4.64-4.70 (m, 1H), 3.22-3.29 (m, 2H), 2.55 (s, 6H), 2.54 (dd, J = 31.2, 15.2 Hz, 4H), 2.17-2.22 (m, 1H), 2.11 (s, 2H), 2.03 (s, 3H), 2.02 (s, 3H), 1.91-2.01 (m, 7H), 1.52 (q, J = 12.0 Hz, 1H), 1.07 (s, 3H), 0.89 (d, J = 6.4 Hz, 3H).

### Example 11 The preparation of compound X

Compound I (10.00mg, 0.22m mol) obtained through example 3 was dissolved in 10mL ethyl acetate, added anhydrous *p*-toluenesulfonic acid (38mg, 0.22mmol), and then stirred at 25°C for half an hour. The solution was evaporated under reduced pressure (0.03∼0.1MPa) at 40°C to afford the *p*-toluenesulfonate (compound IX) (138mg, 0.22mmol).
¹H NMR(400 MHz, (CD3)2SO) of compound IX: 8.92 (s, 1H), 7.49 (d, J = 8.0 Hz, 2H), 7.12 (d, J = 8.0 Hz, 2H), 5.01-5.09 (m, 2H), 4.91-4.94 (m, 1H), 4.66-4.72 (m, 1H), 3.47-3.53 (m, 1H), 3.33-3.40 (m, 1H), 2.81-2.88 (m, 4H), 2.73 (s, 3H), 2.63 (q, J = 10.0 Hz, 1H), 2.29 (s, 3H), 2.17-2.20 (m, 1H), 2.04 (s, 3H), 2.02 (s, 3H), 1.83-1.98 (m, 7H), 1.41 (q, J = 11.6 Hz, 1H), 1.09 (s, 3H), 0.88 (d, J = 5.6 Hz, 3H).

### Example 12 The preparation of compound XI

Compound I (10.00mg, 0.22m mol) obtained through example 3 was dissolved in 10mL ethyl acetate, added anhydrous succinic acid (26mg, 0.22mmol), and then stirred at 25°C for half an hour. The solution was evaporated under reduced pressure (0.03∼0.1MPa) at 40°C to afford the succinate (compound IX) (126mg, 0.22mmol).

### Example 13 The preparation of compound IV form A

Compound IV obtained through example 5 was dissolved in 20mL ethyl acetate, added 50mL hexane and stirred until a lot of crystals deposited. The crystals were filtrated and dried in vacuum at 40 °C to get form A of compound IV (8.5g).

### Example 14 The preparation of compound IV form D

Compound IV obtained through example 5 was put into 30mL MTBE (methyl tert-butyl ether) containing 0.2% water, then stirred for 3 hours at 25°C, The solvent was removed by filtration and the solid was dried at 45°C under 0.03-0.1MPa for 4 hours to ger form D of compound IV (9.1g).

### Example 15 The preparation of compound IV form F

Compound IV obtained through example 5 was dissolved in 20mL methanol. Then 50mL ether were added and stirred until a lot of crystals deposited. The crystals were filtrated and dried at 40 °C under 0.03-0.1MPa for 4 hours to get form F of compound IV(8.9g).

### Example 16 The preparation of compound IV form G

Compound I (10.g) was dissolved in 20mL ethanol, then added with equivalent fumaric acid(2.6g) when stirring. The mixture was stirred for 2 hours and then added with 20mL MTBE to seperate out crystals. The crystals were filtrated , drip washed by MTBE and dried 40°C in vacuum for 2 hours to get form G of compund IV (10.6g).

### Example 17 Pharmacodynamics studies of compound I on EAE model

Mice with experimental autoimmune encephalomyelitis (EAE) model is an ideal animal model of multiple sclerosis (MS), often used in the study of the mechanism of immune activation and immunosuppression.

### 17.1 Induction of MOG-EAE and treatment protocol

Female C57BL/6 mice (six mice per group, 18-20g) received a subcutaneous injection of 300µg MOG 35-55 peptide emulsified in complete Freund's adjuvant containing 400µg Mycobacteria tuberculosis . On Days 0 and 2 post immunization, 250 ng pertussis toxin were applied by intraperitoneal injection.

Mice were weighted and scored for clinical signs daily according to a 5-scale score as described in table 1.

**Table 1. Clinical Score Criteria**

| **Score** | **Description** |
|---|---|
| 0 | Normal mouse; no overt signs of disease |
| 1 | Limp tail or hind limb weakness but not both |
| 2 | Limp tail and hind limb weakness |
| 3 | Partial hind limb paralysis |
| 4 | Complete hind limb paralysis |
| 5 | Moribund state; death by EAE: sacrifice for humane reasons. |

### 17.2 Compound dosing

Compound I was suspended in the 0.5% CMC - Na (carboxymethyl cellulose sodium) and applied in a preventive setting starting from day 0 to day 30 post injection at a dosage of 30 mg/kg body weight once a day via oral gavag. Control animals received vehicle orally as sham treatment.

### 17.3 The results

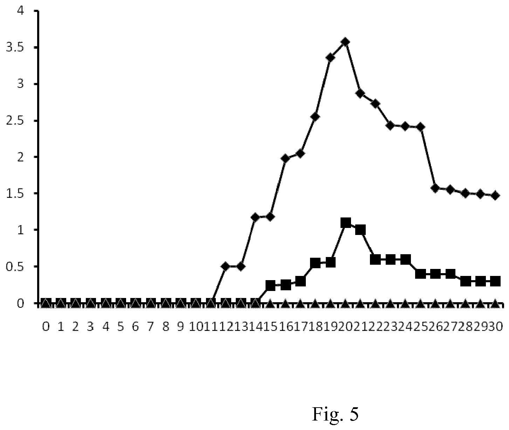

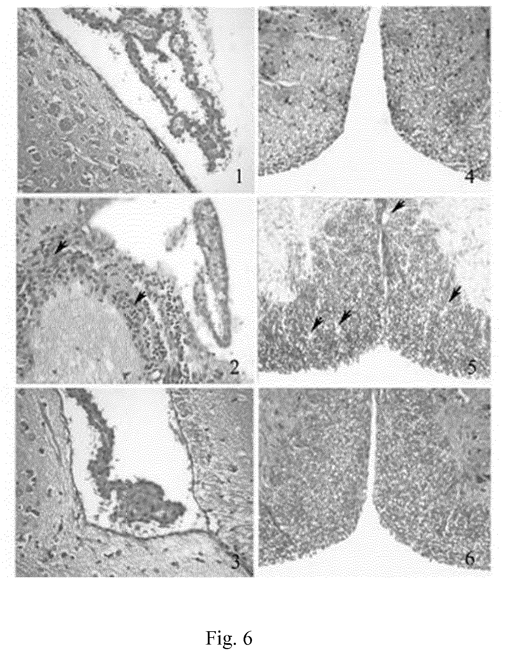

Compound I (30 mg/kg) delayed the onset of EAE by 3 days, and reduced disease severity (Fig. 5). Mice in EAE model group showed inflammation cells infiltration, the arrangement of the organization is loose, and the edge is not clear. Mice treated by compound 1 showed the reducing inflammatory cells, the arrangement of the organization basically returned to normal condition (Fig. 6).

The above experiment repeated 3 times. The results were as same as above mentioned.

### 17.4 Toxicity

Mice were divided into four groups for each having three males and three females and treated by compound 1 at a single-dose of 200, 600, 1800 and 5400 mg/kg, respectively. Five mice except one male mouse died in the group of 5400mg/kg. One female mouse died in the group treated at dose of 1800mg/kg. No obvious toxic reactions were observed in groups treated at dose of 200 and 600mg/kg . The results indicated that compound 1 had a good safety.

### 17.5 The conclusion

Compound 1 (30mg/kg) ameliorated the course of EAE, which indicated that it might have good effect on MS and have potential in preparation of drug for treatment of MS.

### Example 18 Pharmacodynamics studies of the fumarate of dimethylamino 4-O-acetyl LA (compound IV) on EAE model 18.1 Induction of MOG-EAE and treatment protocol

The induction method was the same with example 17.

### 18.2 Compound dosing

Compund IV was applied in a preventive setting starting from Day 0 post injection at a dosage of 30 mg/kg body weight once a day via oral gavag. Control animals received vehicle orally as sham treatment.

### 18.3 Results

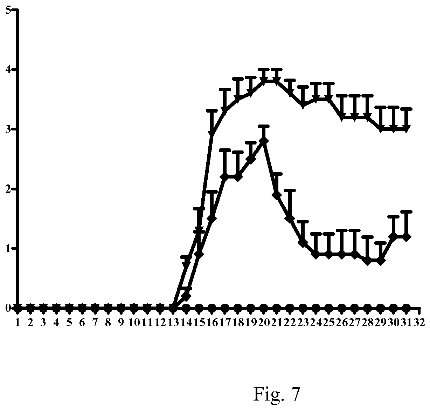

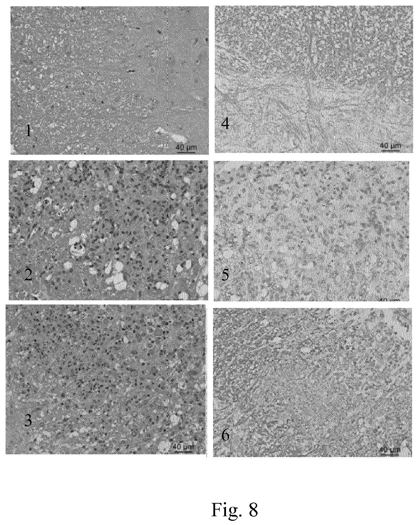

Compound IV(15mg/kg) delayed the onset of EAE and reduced disease severity (Fig. 7). Mice in EAE model group showed inflammation cells infiltration, the arrangement of the organization is loose, and the edge is not clear. Mice treated by compound IV showed the reducing inflammatory cells, the arrangement of the organization basically returned to normal condition and significant lower demyelination comparing to EAE model control (Fig. 8).

### 18.4 Toxicity of Compound IV

Sprague Dawley rats, each group having 3 males and 3 females were orally administrated daily at 75, 150, 300mg/kg respectively for 7 consecutive days. No obvious toxic reactions were observed which indicated that compound IV had a good safety.

### 18.5 Conclusion

Compound IV ameliorated the course of EAE, which indicated that it might have good effect on MS and have potential in preparation of drug for treatment of MS.

### Example 19 Preparation of tablets

| | |
|---|---|
| Compound I | 25 g |
| Lactose | 210 g |
| Corn starch | 60 g |
| Magnesium stearate | 5 g |

Preparation: Mix compound I with lactose and corn starch, granulate with water following with drying ,sieving, mixing with magnesium stearate and tablet compressing. The final tablet weight was about 300mg for each containing 25 mg of compound I .

### Example 20: Preparation of injections

| | |
|---|---|
| Compound III | 5 g |
| Glucose | 50 g |

Preparation methods: Compound III and glucose were dissolved in a suitable amount of water for injection, then filtered and filled into infusion bottles under sterile conditions. Each bottle had 100mL solution containing 5 mg compound III.

### Example 21: Preparation of lyophilization for injection

| | |
|---|---|
| Compound IV | 10 g |
| Mannitol | 30 g |

Preparation: Compound IV and glucose were dissolved in a suitable amount of water for injection, then filtered and filled an aliquot of 2mL into 10mL schering bottle under sterile conditions and finally freeze-dried to have 10 mg compound IV for each bottle.

## Claims

1. A derivative of Line leave Inula flower lactone A (LA), dimethylamino 4-O-acetyl LA, having a structure of

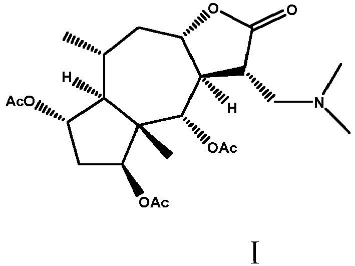

2. The salts of dimethylamino 4-O-acetyl LA applicable in pharmaceuticals, having a common structure of 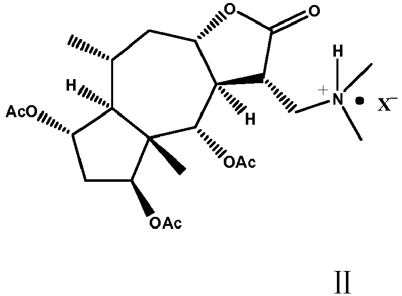 The claimed salts of dimethylamino 4-O-acetyl LA applicable in pharmaceuticals were synthesized by dimethylamino 4-O-acetyl LA and acids (HX).

3. According to the salts of dimethylamino 4-O-acetyl LA applicable in pharmaceuticals that were stated in claim 2, the acids (HX) could be hydrochloric acid (HCl), sulfuric acid (H₂SO₄), hydrofluoric acid (HF), hydrobromic acid (HBr), hydroiodic acid (HI), Phosphoric acid (H₃PO₄), sulfurous acid (H₂SO₃), nitric acid(HNO₃), carbonic acid (H₂CO₃), boric acid (H₃BO₃), seleninic acid (H₂SeO₃), Phosphomolybdic acid(H₃PO4·12MoO₃), phosphorous acid(H₃PO₃), citric acid, maleic acid, D-malic acid, L-malic acid, DL-malic acid, D-lactic acid, L- lactic acid, DL-lactic acid, oxalic acid, sulphonic acid, benzene sulfonic acid, substituted benzene sulfonic acids, 1- naphthalenesulfonic acid, 2-naphthalenesulfonic acid, phthalic acid, tartaric acid, propandioic acid, succinic acid, fumaric acid, orotic acid, benzoic acid or substituted benzoic acids.

4. According to the salts of dimethylamino 4-O-acetyl LA applicable in pharmaceuticals that were stated in claim 2, The optimization of the salts of dimethylamino 4-O-acetyl LA could be hydrochloride (compound III), fumarate (compound IV), nitrate (compound V), hydrobromate (compound VI), sulfurate (compound VII), orotate (compound VIII), citrate (compound IX), p-toluenesulfonate (compound X) or succinate (compound XI), having structures of

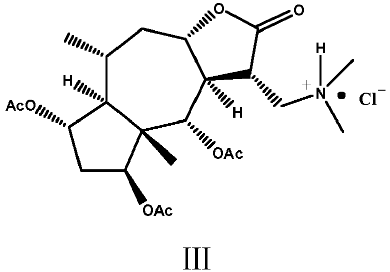

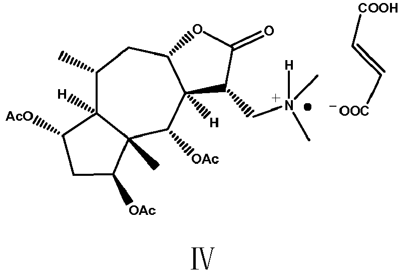

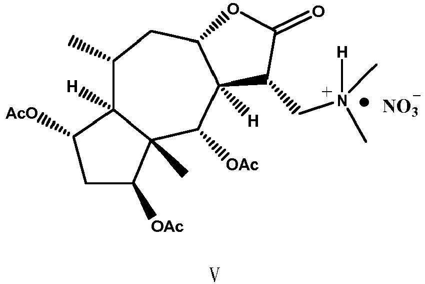

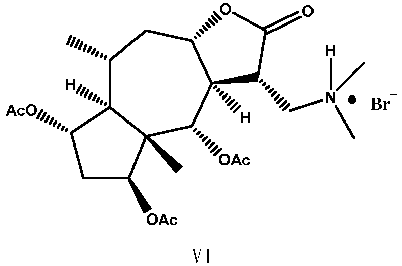

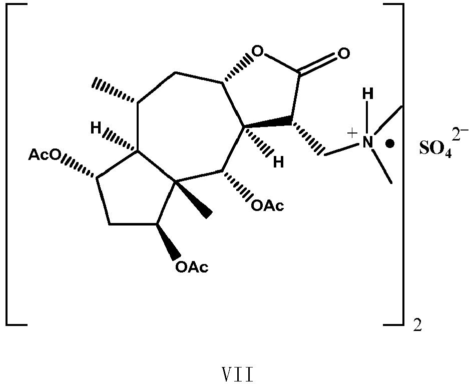

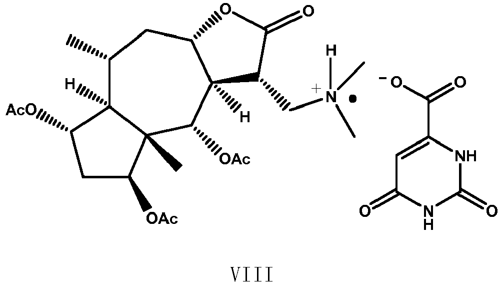

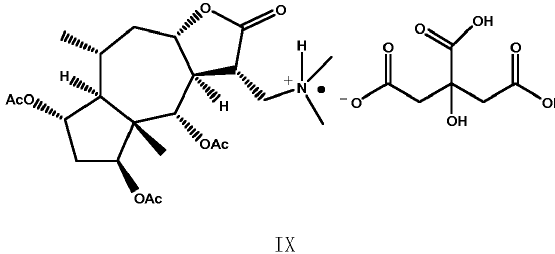

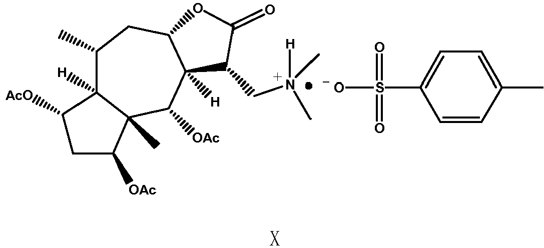

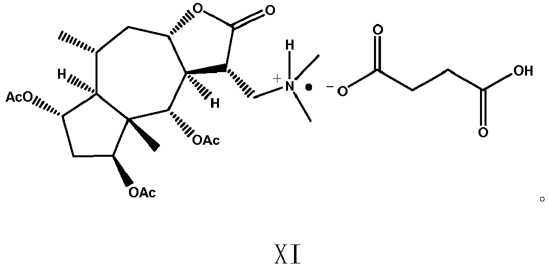

5. According to the salts of dimethylamino 4-O-acetyl LA applicable in pharmaceuticals that were stated in claim 4, the most optimization of the salts of dimethylamino 4-O-acetyl LA were hydrochloride (compound III) and fumarate (compound IV).

6. The preparation methods of compound I and its salts that are applicable in pharmaceuticals were described as follows.
( 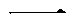 ) The processes to prepare 4-O-acetyl LA were as follows:
(1) The solution of LA, base and acetic anhydride (Ac₂O) in the round-bottom flask was stirred at 0∼50°C for 12∼24h, the reaction was monitored by TLC, until the reaction material was consumed completely. The reaction was quenched by water, and the organic layer was retained.
(2) The organic layer was extracted with water several times until the reaction product 4-O-acetyl LA in the organic phase was pure without other impurities (monitored by TLC), then the aqueous layer was discarded. The organic layer was washed with saturated brine and dried with anhydrous sodium sulfate. Then the solvent was evaporated to 1/10∼1/20 of the original volume under reduced pressure (0.01∼0.1MPa) at 20∼80 °C.
(3) The concentrated solution was purified by silica-gel column chromatography, gradient eluted with PE/EA 15:1∼5:1 to afford 4-O-acetyl LA, monitored by TLC.
Represented as the following equation: 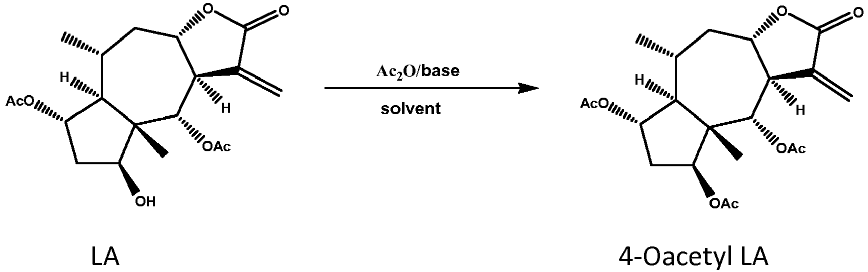
(4) The solution of 4-O-acetyl LA and dimethylamine hydrochloride in the reaction flask was stirred at -20"∼°C, the base was added to the mixture slowly and carefully, then the reaction mixture was warmed up to room temperature (25-30°C) and stirred for 2∼12h, the reaction was monitored by TLC. Until the reaction material was consumed completely, the solvent was evaporated under reduced pressure (0.01∼0.1MPa) at 20∼80 °C to afford the rude product. Then the rude product was dissolved in the organic solvent, extracted with water, the aqueous layer was discarded, the same procedure was repeated several times until the reaction product dimethylamino-4-O-acetyl LA in the organic layer was pure without other impurities (monitored by TLC), The organic layer was washed with saturated brine and dried with anhydrous sodium sulfate. The concentrated solution was purified by silica-gel column chromatography, gradient eluted with PE/EA 15:1∼1:1 to afford the solution of dimethylamino-4-O-acetyl LA, monitored by TLC. Then the solvent was evaporated under reduced pressure (0.01∼0.1MPa) at 20∼80°C to afford dimethylamino-4-O-acetyl LA (compound I)
Represented as the following equation: 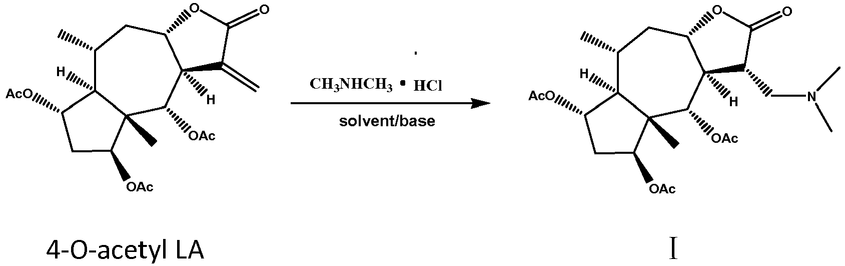
( 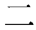 ) The preparation method of salt of dimethy-4-O-acetyl LA was as follows:
The solution of compound I and acid (HX) was stirred at 0∼50°C for 2∼24h, then the solvent was evaporated under reduced pressure (0.01∼0.1MPa) at 20∼80°C to afford the salt of compound II
Represented as the following equation:

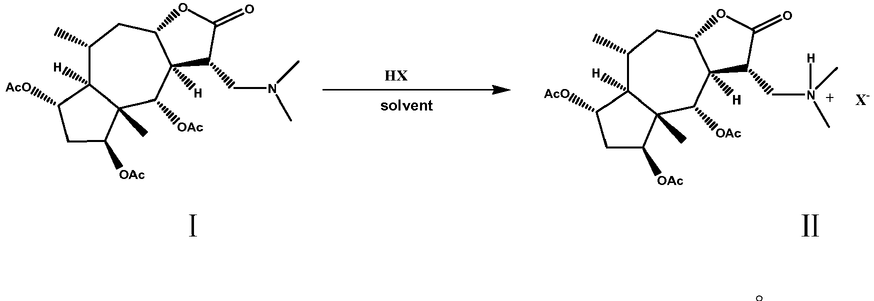

7. According to claim 6, the preparation methods of compound I and its salts that are applicable in pharmaceuticals, wherein the ratio of LA, base and acetic anhydride (Ac₂O) was 1.0eq: 1.0∼2.0eq: 1.0∼2.0eq in the first step of preparation of compound I .
The mentioned organic reagent was one or several mixtures of ethyl acetate, dichloromethane, chloroform, tetrachloromethane, diethyl ether, petroleum ether, n-butyl alcohol, benzene, methylbenzene or tetrahydrofuran. Dichloromethane was preferred.
The mentioned base was low molecular weight trialkyl amine, which was selected from trimethylamine, triethylanmine, tripropylamine, tributylamine, pyridine or replaced pyridine. 2,6-Dimethylpyridine was preferred.
The ratio of 4-O-acetyl LA, base and acetic anhydride (Ac₂O) was 1.0eq: 1.0∼2.0eq: 1.0∼2.0eq in the fourth step. The mentioned organic solvent was one or several mixtures of low molecular weight fatty alcohols, which was selected from methanol, ethanol, propanol, isopropanol, dichloromethane, chloroform, tetrachloromethane, diethyl ether, petroleum ether or ethyl acetate. Ethanol was preferred.
The mentioned organic reagent was one or several mixtures of ethyl acetate, dichloromethane, chloroform, tetrachloromethane, diethyl ether, petroleum ether, n-butyl alcohol, benzene, methylbenzene or tetrahydrofuran. Dichloromethane was preferred.
The mentioned base was low molecular weight trialkylamine, pyridine or the replaced pyridine. The alkyl groups were methyl, ethyl, propyl and butyl. Triethylamine was preferred.

8. According to claim 6, the preparation methods of compound I and its salts that are applicable in pharmaceuticals, wherein the ratio of dimethy-4-O-acetyl LA (compound I) and acid (HX) was 1.0eq: 1.0∼1.5eq in the preparation of the salts of compound I .
The mentioned HX was hydrochloric acid (HCl), sulfuric acid (H₂SO₄), hydrofluoric acid (HF), hydrobromic acid (HBr), hydroiodic acid (HI), Phosphoric acid (H₃PO₄), sulfurous acid (H₂SO₃), nitric acid(HNO₃), carbonic acid (H₂CO₃), boric acid (H₃BO₃), seleninic acid(H₂SeO₃), Phosphomolybdic acid (H₃PO4·12MoO₃), phosphorous acid(H₃PO₃), citric acid, maleic acid, D-malic acid, L-malic acid, DL-malic acid, D-lactic acid, L- lactic acid, DL- lactic acid, oxalic acid, sulphonic acid, benzene sulfonic acid, substituted benzene sulfonic acids, 1- naphthalenesulfonic acid, 2-naphthalenesulfonic acid, phthalic acid, tartaric acid, propandioic acid, butanedioic acid, fumaric acid, orotic acid, benzoic acid or substituted benzoic acids. hydrochloric acid and fumaric acid were preferred.
The mentioned solvent was one or several mixtures of low molecular weight fatty alcohols, which was selected frommethanol, ethanol, propanol, isopropanol, dichloromethane, chloroform, tetrachloromethane, diethyl ether, petroleum ether or ethyl acetate. Ethanol was preferred.

9. A crystal form A of the fumarate of dimethylamino-4-O-acetyl LA (compound IV).
The mentioned crystal form A of compound IV was characterised using PXRD (powder X-ray diffraction) with the values of 2θ, d and relative hights listed in the following table .
| 2θ | d(Ǻ) | I(Height)% |
|---|---|---|
| 8.101 | 10.9048 | 5.6 |
| 9.72 | 9.092 | 66.5 |
| 10.22 | 8.6484 | 77.4 |
| 11.62 | 7.6093 | 37.7 |
| 12.381 | 7.1435 | 6.8 |
| 14.86 | 5.9568 | 11.7 |
| 15.84 | 5.5903 | 37.8 |
| 16.28 | 5.4402 | 32.3 |
| 17.499 | 5.0639 | 18.4 |
| 19.56 | 4.5347 | 70.2 |
| 20.64 | 4.2998 | 100 |
| 21.081 | 4.2108 | 21.9 |
| 21.819 | 4.07 | 42.6 |
| 23.381 | 3.8016 | 72.4 |
| 24.12 | 3.6866 | 4.3 |
| 24.939 | 3.5674 | 41.5 |
| 25.281 | 3.52 | 40.3 |
| 26.34 | 3.3808 | 7.5 |
| 26.901 | 3.3115 | 11.4 |
| 27.581 | 3.2315 | 16.4 |
| 29.436 | 3.0318 | 4.8 |
| 30.021 | 2.9741 | 7.2 |
| 31.001 | 2.8823 | 23.4 |
| 32.038 | 2.7913 | 6.9 |
| 32.498 | 2.7528 | 2.3 |
| 32.922 | 2.7183 | 3.2 |
| 33.302 | 2.6882 | 10.1 |
| 34.361 | 2.6077 | 3.2 |
| 35.879 | 2.5008 | 5.9 |
| 37.34 | 2.4062 | 5.5 |
| 38.998 | 2.3077 | 4.3 |
| 40.121 | 2.2456 | 7.5 |
| 41.8 | 2.1592 | 7.4 |
The PXRD pattern of form A was shown in Fig. 1

10. A method of preparation of the crystal form A of compound IV, wherein in the first step, compound IV was dissolved in 2 to 5 folds ethyl acetate. Then 2 to 5 folds hexane were added and stirred until a lot of crystals deposited. The crystals were filtrated and dried in vacuum at 25∼40°C to get form A of compound IV.

11. A crystal form D of the fumarate of dimethylamino-4-O-acetyl LA (compound IV).
The mentioned crystal form D of compound IV was characterised using PXRD (powder X-ray diffraction) with the values of 2θ, d and relative hights listed in the following table .
| 2θ | d(Ǻ) | I(Height)% |
|---|---|---|
| 8.6 | 10.2736 | 100 |
| 9.777 | 9.0388 | 30.8 |
| 10.659 | 8.2929 | 21.5 |
| 11.102 | 7.9633 | 3.7 |
| 13.021 | 6.7936 | 10 |
| 14.64 | 6.0457 | 60.5 |
| 15.039 | 5.8861 | 24 |
| 16.24 | 5.4535 | 35.4 |
| 16.879 | 5.2484 | 20 |
| 17.298 | 5.1221 | 4.7 |
| 17.8 | 4.979 | 5.5 |
| 18.659 | 4.7514 | 13.4 |
| 19.12 | 4.638 | 16.1 |
| 19.619 | 4.5211 | 78.2 |
| 21.418 | 4.1453 | 10.6 |
| 22.3 | 3.9832 | 42.2 |
| 22.801 | 3.8969 | 10.3 |
| 23.266 | 3.82 | 3.8 |
| 23.939 | 3.7141 | 50.4 |
| 24.699 | 3.6015 | 35.6 |
| 25.259 | 3.5229 | 9.7 |
| 25.639 | 3.4716 | 8.3 |
| 26.24 | 3.3935 | 20.8 |
| 27.24 | 3.2711 | 19 |
| 27.961 | 3.1883 | 8.8 |
| 28.379 | 3.1423 | 4.2 |
| 28.659 | 3.1123 | 15.7 |
| 28.981 | 3.0784 | 12.7 |
| 29.62 | 3.0135 | 14.2 |
| 29.941 | 2.9818 | 6.5 |
| 30.36 | 2.9416 | 11.2 |
| 30.996 | 2.8827 | 7.4 |
| 31.478 | 2.8397 | 4 |
| 31.861 | 2.8064 | 11.5 |
| 35.799 | 2.5062 | 4.6 |
| 36.646 | 2.4502 | 4.7 |
| 39.882 | 2.2586 | 7.3 |
| 41.057 | 2.1966 | 9.5 |
| 41.9 | 2.1543 | 4.5 |
The PXRD pattern of form A was shown in Fig. 2

12. A method of preparation of the crystal form D of compound IV, wherein in the first step, Compound IV was put into MTBE (methyl tert-butyl ether) containing 0.2% water, then stirred for 2-5 hours. The solvent was removed by filtration and the solid was dried at 40-60°C under 0.03-0.1MPa for 4-24 hours to ger form D of compound IV.

13. A crystal form F of the fumarate of dimethylamino-4-O-acetyl LA (compound IV).
The mentioned crystal form F of compound IV was characterised using PXRD (powder X-ray diffraction) with the values of 2θ, d and relative hights listed in the following table .
| 2θ | d(Ǻ) | I(Height)% |
|---|---|---|
| 8.06 | 10.9609 | 5.4 |
| 9.659 | 9.1493 | 100 |
| 10.08 | 8.7682 | 91.7 |
| 11.5 | 7.6884 | 42.3 |
| 12.139 | 7.2851 | 7.7 |
| 14.642 | 6.0449 | 9.8 |
| 15.639 | 5.6616 | 35.8 |
| 16.2 | 5.4669 | 30 |
| 17.441 | 5.0806 | 15.1 |
| 19.48 | 4.5531 | 73 |
| 20.44 | 4.3413 | 68.1 |
| 20.679 | 4.2916 | 99.4 |
| 21.639 | 4.1034 | 40.1 |
| 23.28 | 3.8177 | 54.9 |
| 24.68 | 3.6043 | 21.8 |
| 25.261 | 3.5227 | 29.8 |
| 25.938 | 3.4322 | 6 |
| 26.52 | 3.3582 | 11.3 |
| 26.938 | 3.3071 | 4.3 |
| 27.482 | 3.2428 | 9.8 |
| 27.958 | 3.1887 | 4.4 |
| 29.201 | 3.0557 | 5.1 |
| 29.918 | 2.9841 | 7.3 |
| 30.442 | 2.9339 | 8.6 |
| 30.839 | 2.8971 | 13.4 |
| 31.657 | 2.824 | 4.7 |
| 33.3 | 2.6884 | 6.2 |
| 34.341 | 2.6092 | 2.6 |
| 35.02 | 2.5601 | 4.1 |
| 35.778 | 2.5076 | 4.3 |
The PXRD pattern of form A was shown in Fig. 3.

14. A method of preparation of the crystal form F of compound IV, wherein in the first step, compound IV was dissolved in 1 to 2 folds methanol. Then 2 to 5 folds ether were added and stirred until a lot of crystals deposited. The crystals were filtrated and dried at 25∼40°C under 0.03-0.1MPa to get form F of compound IV.

15. A crystal form G of the fumarate of dimethylamino-4-O-acetyl LA (compound IV).
The mentioned crystal form G of compound IV was characterised using PXRD (powder X-ray diffraction) with the values of 2θ, d and relative hights listed in the following table .
| 2θ | d(Ǻ) | I(Height)% |
|---|---|---|
| 8.567 | 10.31279 | 100.0 |
| 7.931 | 9.08148 | 17.0 |
| 10.849 | 8.14803 | 16.7 |
| 11.163 | 7.92006 | 193.4 |
| 12.452 | 7.10295 | 0.8 |
| 12.865 | 6.87584 | 2.7 |
| 13.115 | 6.74542 | 1.0 |
| 14.413 | 6.14053 | 17.9 |
| 14.869 | 5.95311 | 20.0 |
| 15.128 | 5.85184 | 3.2 |
| 16.225 | 5.45870 | 9.0 |
| 17.268 | 5.13128 | 13.8 |
| 18.761 | 4.72601 | 26.8 |
| 19.661 | 4.51181 | 6.0 |
| 21.872 | 4.06030 | 20.3 |
| 22.059 | 4.02635 | 22.8 |
| 23.059 | 3.85401 | 1.9 |
| 23.631 | 3.73193 | 3.0 |
| 24.014 | 3.70281 | 2.1 |
| 24.809 | 3.58595 | 3.0 |
| 25.401 | 3.50361 | 13.1 |
| 25.830 | 3.44652 | 6.9 |
| 26.245 | 3.39285 | 7.6 |
| 27.267 | 3.26804 | 2.5 |
| 28.160 | 3.16634 | 5.4 |
| 29.131 | 3.06300 | 5.0 |
| 30.138 | 2.96286 | 3.8 |
| 30.797 | 2.90094 | 4.1 |
| 32.824 | 2.72631 | 1.0 |
| 33.257 | 2.69179 | 0.6 |
| 34.654 | 2.58642 | 1.6 |
| 36.124 | 2.48445 | 1.2 |
| 38.027 | 2.36438 | 1.0 |
| 38.503 | 2.33628 | 1.0 |
| 38.942 | 2.31091 | 1.0 |
The PXRD pattern of form A was shown in Fig. 4.

16. A method of preparation of the crystal form G of compound IV, wherein in the first step,compound I was dissolved in 2 folds ethanol, then added with equivalent fumaric acid when stirring. The mixture was stirred for 0.5-2 hours and then added with 1 to 2 folds MTBE to seperate out crystals. The crystals were filtrated , drip washed by MTBE and dried 25∼40°C in vacuum to get form G of compund IV.

17. A method for treating multiple sclerosis by administrating dimethylamino-4-O-acetyl LA or its salts applicable in pharmaceuticals.

18. A method of treating multiple sclerosis by administrating a composition of dimethylamino-4-O-acetyl LA(compound I) or its salts applicable in pharmaceuticals(compound II) combined with medicinal carriers.

19. The method for treating multiple sclerosis according to claim 18, wherein the pharmaceutical composition is administrated by tablets, dispersible tablets, mouth collapse tablets, retard tablets, capsules, soft capsules, dropping pills, granules, injections, powder injections or aerosols.
